# EUROPÄISCHE PATENTANMELDUNG

(11) **EP 4 779 271 A2**
(43) Veröffentlichungstag der Anmeldung: **22.07.2026**
(21) Anmeldenummer: 26151727.0
(22) Anmeldetag: 14.01.2026
(51) Int. Cl.: G01G 17/04, A61M 1/16

(54) **VORRICHTUNG ZUM WIEGEN EINER FLÜSSIGKEITSMENGE IN EINER EXTRAKORPORALEN BLUTBEHANDLUNGSMASCHINE**

(30) Priorität: 15.01.2025 DE 102025101279
(71) Anmelder: B. Braun Avitum AG, 34212 Melsungen (DE)
(72) Erfinder: AHRENS, Joern, 34225 Baunatal (DE)
(74) Vertreter: Winter, Brandl - Partnerschaft mbB

(57) **Zusammenfassung**

Vorrichtung (10) zum Wiegen einer Flüssigkeitsmenge in einer extrakorporalen Blutbehandlungsmaschine, umfassend: eine Befestigungseinheit (11), die eingerichtet ist, einen Behälter (13) für die Flüssigkeitsmenge an einem Gestellelement (14) zu befestigen; eine Wägesensoreinheit (15) mit einem ersten Messkanal (16) und einem zweiten Messkanal (17), wobei die Wägesensoreinheit (15) eingerichtet ist, einen ersten Gewichtsmesswert über den ersten Messkanal (16) zu erfassen und einen zweiten Gewichtsmesswert über den zweiten Messkanal (17) zu erfassen; eine Steuerungseinheit (18), die eingerichtet, auf Basis des erfassten ersten Gewichtsmesswertes und des erfassten zweiten Gewichtsmesswertes ein Gewicht für die Flüssigkeitsmenge in dem Behälter (13) zu bestimmen; wobei die Wägesensoreinheit (15) eingerichtet ist, jeweils vor dem Wiegen der Flüssigkeitsmenge in einem unbelasteten Zustand der Wägesensoreinheit (15) einen ersten Initialisierungsgewichtsmesswert über den ersten Messkanal (16) zu erfassen und einen zweiten Initialisierungsgewichtsmesswert über den zweiten Messkanal (17) zu erfassen (S1); wobei die Steuerungseinheit (18) eingerichtet ist, den erfassten ersten Initialisierungsgewichtsmesswert mit einem ersten Grenzwert zu vergleichen und den zweiten Initialisierungsgewichtsmesswert mit einem zweiten Grenzwert zu vergleichen (S2); wobei die Steuerungseinheit (18) eingerichtet ist: wenn ein Betrag des ersten Initialisierungsgewichtsmesswert kleiner als ein Betrag des ersten Grenzwerts ist und ein Betrag des zweiten Initialisierungsgewichtsmesswerts kleiner als ein Betrag des zweiten Grenzwerts ist, den erfassten ersten Initialisierungsgewichtsmesswert als Offset für den ersten Messkanal (16) festzulegen und den erfassten zweiten Initialisierungsgewichtsmesswert als Offset für den zweiten Messkanal (17) festzulegen (S3); und wenn ein Betrag des ersten Initialisierungsgewichtsmesswerts größer als ein Betrag des ersten Grenzwerts ist und/oder ein Betrag des zweiten Initialisierungsgewichtsmesswerts größer als ein Betrag des zweiten Grenzwerts ist, eine Fehlermeldung auszugeben (S4).

## Beschreibung

### Technisches Gebiet

Die vorliegende Offenbarung betrifft eine Vorrichtung zum Wiegen einer Flüssigkeitsmenge in einer extrakorporalen Blutbehandlungsmaschine, insbesondere einem Dialysegerät, ein computerimplementiertes Verfahren zum Betreiben einer Vorrichtung zum Wiegen einer Flüssigkeit, ein Computerprogrammprodukt, ein computerlesbares Speichermedium sowie ein Dialysegerät mit einer solchen Vorrichtung.

### Technischer Hintergrund

Wägezellen für Dialysegeräte sind aus dem Stand der Technik grundsätzlich bekannt und werden in Dialysegeräten zum Messen einer Flüssigkeitsmenge eingesetzt. Hierbei ist es wichtig, die Flüssigkeitsmengen, die dem Körper entnommen und zugeführt werden, in einem Gleichgewicht zu halten. Falls dies nicht entsprechend genau gelingt, kann es zu Komplikationen beim Patienten kommen. Die Wägezellen unterliegen jedoch einem Verschleiß bzw. verändern ihre Eigenschaften über die Zeit. Aus diesem Grund müssen die Wägezellen von Zeit zu Zeit ausgetauscht und/oder neu kalibriert werden. Dieser Vorgang kann aufgrund von Regularien in der Regel nicht von medizinischen Mitarbeitern durchgeführt werden. Stattdessen sind speziell geschulte Techniker notwendig. Dies birgt hohe Kosten, eine geringere Verfügbarkeit und gegebenenfalls während des Betriebs ungenauere Messergebnisse für die Flüssigkeitsmengen und somit ein Gesundheitsrisiko für Patienten.

In diesem Zusammenhang hat sich nun herausgestellt, dass ein weiterer Bedarf besteht, eine Vorrichtung zum Wiegen einer Flüssigkeitsmenge in einem Dialysegerät bereitzustellen.

### Zusammenfassung der vorliegenden Offenbarung

Es ist daher Aufgabe der vorliegenden Offenbarung eine Vorrichtung zum Wiegen einer Flüssigkeitsmenge in einer extrakorporalen Blutbehandlungsmaschine, insbesondere einem Dialysegerät, bereitzustellen, insbesondere ist es Aufgabe der vorliegenden Offenbarung, eine effiziente und genaue Vorrichtung zum Wiegen einer Flüssigkeitsmenge in einer extrakorporalen Blutbehandlungsmaschine bereitzustellen.

Die Aufgabe der vorliegenden Offenbarung wird durch eine Vorrichtung zum Wiegen einer Flüssigkeitsmenge in einer extrakorporalen Blutbehandlungsmaschine gemäß Anspruch 1, sowie durch die nebengeordneten Ansprüche gelöst. Vorteilhafte Ausgestaltungen sind Gegenstand der Unteransprüche und/oder werden nachfolgend erläutert.

Ein erster Aspekt der vorliegenden Offenbarung betrifft eine Vorrichtung zum Wiegen einer Flüssigkeitsmenge in einer extrakorporalen Blutbehandlungsmaschine, umfassend: eine Befestigungseinheit, die eingerichtet ist, einen Behälter, beispielsweise Beutel, für die Flüssigkeitsmenge an einem Gestellelement zu befestigen, eine Wägesensoreinheit mit einem ersten Messkanal und einem zweiten Messkanal, wobei die Wägesensoreinheit eingerichtet ist, einen ersten Gewichtsmesswert über den ersten Messkanal zu erfassen und einen zweiten Gewichtsmesswert über den zweiten Messkanal zu erfassen, eine Steuerungseinheit, die eingerichtet ist, auf Basis des erfassten ersten Gewichtsmesswertes und des erfassten zweiten Gewichtsmesswertes ein Gewicht für die Flüssigkeitsmenge in dem Behälter zu bestimmen, wobei die Wägesensoreinheit eingerichtet ist, jeweils vor dem Wiegen der Flüssigkeitsmenge in einem unbelasteten Zustand der Wägesensoreinheit einen ersten Initialisierungsgewichtsmesswert über den ersten Messkanal zu erfassen und einen zweiten Initialisierungsgewichtsmesswert über den zweiten Messkanal zu erfassen (S1); wobei die Steuerungseinheit eingerichtet ist, den erfassten ersten Initialisierungsgewichtsmesswert mit einem ersten Grenzwert zu vergleichen und den zweiten Initialisierungsgewichtsmesswert mit einem zweiten Grenzwert zu vergleichen (S2); wobei die Steuerungseinheit eingerichtet ist, wenn ein Betrag des ersten Initialisierungsgewichtsmesswerts kleiner als ein Betrag des ersten Grenzwerts ist und ein Betrag des zweiten Initialisierungsgewichtsmesswerts kleiner als ein Betrag des zweiten Grenzwerts ist, den erfassten ersten Initialisierungsgewichtsmesswert als Offset für den ersten Messkanal festzulegen und den erfassten zweiten Initialisierungsgewichtsmesswert als Offset für den zweiten Messkanal festzulegen (S3); und wobei die Steuerungseinheit eingerichtet ist, wenn ein Betrag des ersten Initialisierungsgewichtsmesswerts größer als ein Betrag des ersten Grenzwerts ist und/oder ein Betrag des zweiten Initialisierungsgewichtsmesswerts größer als ein Betrag des zweiten Grenzwerts ist, eine Fehlermeldung auszugeben (S4).

Der Begriff extrakorporale Blutbehandlungsmaschine meint vorliegend insbesondere ein Gerät zur Durchführung einer extrakorporalen Blutbehandlung. Ein Dialysegerät ist ein Beispiel für eine extrakorporale Blutbehandlungsmaschine. Die Dialyse umfasst dabei die Reinigung des Blutes eines Patienten. Die Reinigung umfasst dabei unter anderem die Entfernung von Abfallstoffen, überschüssiger Flüssigkeit und Giftstoffe aus dem Blut. Vorliegend wird insbesondere ein Dialysegerät zur Hämodialyse betrachtet. Das Dialysegerät kann neben der Hämodialyse auch zur Hämofiltration, zur Hämodiafiltration, zur Ultrafiltration, etc. eingerichtet sein. Das Dialysegerät umfasst dabei Pumpen, Sensoren, einen Dialysator sowie Behälter bzw. Beutel, etwa Flüssigkeitsbehälter bzw. Infusionsbehälter.

Der Begriff Befestigungseinheit meint vorliegend ein strukturelles Bauteil, welches eingerichtet ist, einen Behälter, insbesondere Beutel, zu befestigen. Die Befestigungseinheit kann ein Kragarm sein, an dem ein Haken zur Aufnahme für einen Behälter angebracht ist. Die Befestigungseinheit kann eine Schnittstelle zum Befestigen eines Behälters aufweisen. Die Schnittstelle kann einen Haken umfassen. Die Befestigungseinheit kann einteilig oder mehrteilig sein.

Der Begriff Behälter meint vorliegend insbesondere einen Hohlkörper, in den eine Flüssigkeit reinfließen und/oder rausfließen kann. Der Behälter kann eine steife Gehäusewand oder eine flexible Gehäusewand aufweisen. Der Behälter kann einen Beutel umfassen bzw. als ein Beutel ausgebildet sein. Der Behälter kann als Material Kunststoff umfassen. Der Behälter kann ein Flüssigkeitsbehälter, etwa ein Infusionsbehälter oder ein Dialysatbehälter sein.

Der Begriff Gestellelement meint ein strukturelles Bauteil, das eingerichtet ist, eine Befestigungseinheit anzuordnen bzw. aufzunehmen. Das Gestellelement kann beispielsweise ein Ständer sein. Das Gestellelement kann Teil der extrakorporalen Blutbehandlungsmaschine, insbesondere des Dialysegeräts bzw. Dialysesystems sein.

Der Begriff Wägesensoreinheit meint vorliegend eine Einheit mit zumindest zwei Messkanälen zur Erfassung eines Gewichts, wobei unter einem Gewicht gemäß der vorliegenden Offenbarung eine Masse zu verstehen ist. Der Messkanal umfasst dabei einen Sensor, der eingerichtet ist, ein Gewicht zu messen. Der Sensor kann dabei eines oder mehr der folgenden umfassen: DMS Sensor, kapazitiver Sensor, piezoelektrischer Sensor. Der Messkanal umfasst dabei weiterhin Hardware und Software zur Verarbeitung und Weitergabe des erfassten Messwertes. Der erste und zweite Messkanal sind unabhängig voneinander. Der erste und zweite Messkanal können gleich oder unterschiedlich aufgebaut sein. Vorzugsweise sind der erste und zweite Messkanal unterschiedlich aufgebaut.

Der Begriff Grenzwert meint vorliegend einen vorher festgelegten Wert, der nicht überschritten werden darf. Der Grenzwert kann dabei spezifisch für den jeweiligen ersten oder zweiten Messkanal festgelegt werden. Der erste Grenzwert und der zweite Grenzwert können gleich oder verschieden sein. Vorzugsweise sind der erste Grenzwert und der zweite Grenzwert verschieden. Der erste Grenzwert und der zweite Grenzwert können beispielsweise + - 20g betragen. Der erste Grenzwert und der zweite Grenzwert geben eine maximale Anpassungsgröße des Offsets vor. Beispielsweise darf der Offset vorliegend im Rahmen einer neuen Anpassung nicht um 24g in eine Richtung verändert werden. Beispielsweise dürfte der Offset vorliegend im Rahmen einer neuen Anpassung um 17g verändert werden.

Der Begriff Offset meint vorliegend einen Messwert eines Messkanals der Wägesensoreinheit in einem unbelasteten Zustand.

Die Steuerungseinheit kann beispielsweise ein Gewicht für die Flüssigkeitsmenge in dem Behälter dadurch bestimmen, indem sie den ersten Gewichtsmesswert als Wert für das Gewicht für die Flüssigkeitsmenge in dem Behälter verwendet. Die Steuerungseinheit kann beispielsweise ein Gewicht für die Flüssigkeitsmenge in dem Behälter dadurch bestimmen, indem sie einen Mittelwert aus dem ersten Gewichtsmesswert und dem zweiten Gewichtsmesswert als Wert für das Gewicht für die Flüssigkeitsmenge in dem Behälter verwendet. Die Steuerungseinheit kann weiterhin eingerichtet sein, eine Differenz zwischen dem ersten Gewichtsmesswert und dem zweiten Gewichtsmesswert zu bilden und diese mit einem Arbeitsgrenzwert zu vergleichen. Der Arbeitsgrenzwert kann beispielsweise 10g betragen. Die Steuerungseinheit kann weiterhin eingerichtet sein, wenn die Differenz den Arbeitsgrenzwert überschreitet, eine Fehlermeldung (z.B. zu hohe Messabweichung zwischen den Messkanälen) auszugeben. Die Steuerungseinheit kann weiterhin eingerichtet sein, wenn die Differenz den Arbeitsgrenzwert nicht überschreitet, das Gewicht für die Flüssigkeitsmenge in dem Behälter auszugeben.

Der Offenbarung liegt die Erkenntnis zugrunde, dass eine Wägezelle beim Wiegen einer Flüssigkeitsmenge naturgemäß einen Offset aufweist. Das heißt auch in einem unbelasteten Zustand zeigt ein Messkanal der Wägezelle einen Messwert an. Dieser Offset kann sich unter anderem aufgrund von Verschleiß, Veränderung der Umgebungsbedingungen, einem falschen Aufbau der Vorrichtung zum Wiegen der Flüssigkeitsmenge (z.B. falscher Haken oder zusätzliche Last) ändern. Dieser Offset wird im Regelfall mit einem Grenzwert verglichen und sofern der Offset unterhalb des Grenzwerts ist, wird ohne Kalibrierung mit dem alten ursprünglich festgelegten Offset gemessen. Der alte ursprünglich festgelegte Offset stammt dabei aus einer Kalibrierung im Herstellungswerk oder aus einer Inbetriebnahme mit einem zertifizierten Messtechniker. Bei Überschreiten des Grenzwertes wird eine Fehlermeldung ausgegeben. Dies hat jedoch den Nachteil, dass zum einen ungenau gemessen wird und zum anderen schleichende Veränderungen der Umgebungsbedingungen überbewertet werden. Die Erfindung schlägt nun vor, einen Initialisierungsgewichtsmesswert in unbelasteten Zustand vor jeder Messung jeweils für den ersten und zweiten Messkanal zu erfassen und diesen mit einem Grenzwert zu vergleichen und für den Fall, dass dieser Initialisierungsgewichtsmesswert unterhalb des Grenzwertes liegt, diesen als neuen Offset zu verwenden Dies entspricht einer neuen Kalibrierung, bei der jedoch kein Messtechniker notwendig ist. Bei Überschreiten eines Messkanals oder beider Messkanäle wird eine Fehlermeldung ausgegeben. Auf diese Weise kann zum einen die Messgenauigkeit erhöht werden, da jeweils mit einem genaueren Offset gearbeitet wird und zum anderen die Verfügbarkeit der Wägezelle erhöht werden, da eine Fehlermeldung bei einer schleichenden Verschlechterung durch eine neue Kalibrierung vermieden wird. Weiterhin ist es möglich, durch die beschriebene Kalibrierung vor jedem Messdurchlauf den ersten und/oder zweiten Grenzwert größer festzulegen. Dies kann sich ebenfalls vorteilhaft auf die Verfügbarkeit auswirken.

Gemäß einer bevorzugten Ausführungsform kann die Fehlermeldung eine Aufforderung zur Überprüfung der Vorrichtung hinsichtlich von Messbedingungen umfassen.

Die Fehlermeldung kann beispielsweise an einem Display der Vorrichtung angezeigt werden. Die Messbedingungen können den Messaufbau und/oder die Umgebungsbedingungen (z. B. Temperatur, Raumfeuchte, Sonneneinstrahlung) umfassen. Die Fehlermeldung kann beispielsweise Hinweise und/oder Fragen beinhalten, die sich auf den Messaufbau richten. Fragen können beispielsweise umfassen, ob wirklich kein Gewicht an der Vorrichtung hängt oder ob die Vorrichtung bzw. der Haken der Vorrichtung freihängend ist. Der Anwender kann dann dementsprechend die Messbedingungen prüfen und entscheiden, ob die Messung wiederholt werden soll oder ob ein Techniker hingezogen werden soll.

Auf diese Weise kann die Verfügbarkeit der Vorrichtung vorteilhafterweise erhöht werden.

Gemäß einer bevorzugten Ausführungsform kann nach der Überprüfung der Vorrichtung hinsichtlich der Messbedingungen die Vorrichtung eingerichtet sein, die Schritte S1 bis S4 nochmals durchzuführen.

Beispielsweise kann die Vorrichtung weiter eingerichtet sein, nach der Überprüfung eine Bestätigung der Überprüfung durch einen Mitarbeiter zu empfangen. Dies kann beispielsweise über eine Schnittstelle erfolgen, beispielsweise ein Steuerungsfeld, eine Taste oder ein Human Machine Interface (HMI). Die Vorrichtung kann die Schritte S1 bis S4 nochmals ausführen. Bei positivem Ergebnis kann dann erfolgreich gemessen werden. Bei negativen Ergebnis kann beispielsweise eine erneute Fehlermeldung mit demselben Inhalt oder gegebenenfalls ein Hinweis zur Hinzuziehung eines Technikers erfolgen. Letzteres führt dann zu einem unweigerlichen vorläufigen Ausfall der Vorrichtung.

Auf diese Weise kann die Verfügbarkeit der Vorrichtung insgesamt erhöht werden, da die notwendige Inspektion eines Technikers mit neuer aufwändiger Kalibrierung umgangen werden kann.

Gemäß einer bevorzugten Ausführungsform kann die Steuerungseinheit weiter eingerichtet sein, eine erste Differenz zwischen dem ersten erfassten Initialisierungsgewichtsmesswert und einem ersten Ausgangsinitialisierungsgewichtsmesswert mit einem ersten Absolutgrenzwert zu vergleichen, eine zweite Differenz zwischen dem zweiten erfassten Initialisierungsgewichtsmesswert und einem zweiten Ausgangsinitialisierungsgewichtsmesswert mit einem zweiten Absolutgrenzwert zu vergleichen, wenn die erste Differenz kleiner als der erste Absolutgrenzwert und die zweite Differenz kleiner als der zweite Absolutgrenzwert ist, den erfassten ersten Initialisierungsgewichtsmesswert als Offset für den ersten Messkanal festzulegen und den erfassten zweiten Initialisierungsgewichtsmesswert als Offset für den zweiten Messkanal festzulegen, und wenn die erste Differenz größer als der erste Absolutgrenzwert ist und/oder die zweite Differenz größer als der zweite Absolutgrenzwert ist, eine Fehlermeldung auszugeben.

Der Begriff Ausgangsinitialisierungsgewichtsmesswert meint vorliegend insbesondere einen Messwert, der im Rahmen einer Kalibrierung mit Messgewichten, beispielsweise bei der Herstellung der Wägesensoreinheit bzw. der Vorrichtung, ermittelt wurde. Der erste Ausgangsinitialisierungsgewichtsmesswert kann sich dabei vorzugsweise auf den ersten Messkanal beziehen. Der zweite Ausgangsinitialisierungsgewichtsmesswert kann sich dabei vorzugsweise auf den zweiten Messkanal beziehen.

Der Begriff Absolutgrenzwert meint vorliegend einen Grenzwert für eine Abweichung von einem Ausgangsinitialisierungsgewichtsmesswert. Der erste Absolutgrenzwert ist beispielsweise +-100g. Der zweite Absolutgrenzwert ist beispielsweise +-100g. Die beiden Absolutgrenzwerte können gleich oder unterschiedlich sein.

Die Differenz zwischen dem ersten Ausgangsinitialisierungsgewichtsmesswert und dem erfassten ersten Initialisierungsgewichtsmesswert kann dabei vorteilhafterweise Aufschluss darüber geben, wie weit sich der aktuell erfasste Initialisierungsgewichtsmesswert vom ursprünglichen ersten Ausgangsinitialisierungsgewichtsmesswert entfernt. Bei Überschreiten des ersten Absolutgrenzwertes, kann davon ausgegangen werden, dass ein Defekt am Messkanal und/oder keine sinnvollen Messbedingungen vorliegen. Gleiches gilt analog für den zweiten erfassten Initialisierungsgewichtsmesswert und den zweiten Ausgangsinitialisierungsgewichtsmesswert.

Auf diese Weise kann auf synergetische Weise vorteilhaft ein Trend mildernd berücksichtigt werden, aber gleichzeitig eine zu große Veränderung der Messkanäle gegenüber dem Auslieferungszustand berücksichtigt werden. Dies kann die Verfügbarkeit bei gleichzeitiger Verlässlichkeit der Vorrichtung erhöhen.

Gemäß einer bevorzugten Ausführungsform kann die Steuerungseinheit weiter eingerichtet sein, wenn der erste Initialisierungsgewichtsmesswert größer als der erste Grenzwert ist und der zweite Initialisierungsgewichtsmesswert größer als der zweite Grenzwert ist, und wenn eine Differenz des ersten Initialisierungsgewichtsmesswerts und des zweiten Initialisierungsgewichtsmesswerts kleiner als ein Toleranzgrenzwert ist, eine Fehlermeldung mit einer Aufforderung zur Überprüfung der Messbedingungen auszugeben.

Sofern sich beide Initialisierungsgewichtsmesswerte in gleichem Maße verändern, kann davon ausgegangen werden, dass die Ursache an den Messbedingungen liegt und nicht an der Hardware und/oder Software der Messkanäle. Beispielsweise kann es zu einer Temperaturerhöhung oder einer mechanischen Belastung der Vorrichtung gekommen sein. Die Vorrichtung adressiert diesen Umstand, indem sie eine zusätzliche Überprüfung der Initialisierungsgewichtsmesswerte untereinander durchführt und dann eine entsprechende Fehlermeldung ausgibt.

Auf diese Weise kann auf effiziente Weise ein Fehler in den Messbedingungen aufgespürt werden.

Gemäß einer bevorzugten Ausführungsform kann die Steuerungseinheit weiter eingerichtet sein, während dem Betrieb eine Differenz des mit dem ersten Messkanal bestimmten Gewichts und des mit dem zweiten Messkanal bestimmten Gewichts mit einem Betriebsgrenzwert zu vergleichen, und wenn die Differenz größer als der Betriebsgrenzwert ist, eine Fehlermeldung auszugeben.

Der erste Messkanal liefert beispielsweise das Ergebnis für das Gewicht für die Flüssigkeitsmenge. Der zweite Messkanal kann vorzugsweise der Überprüfung des ersten Messkanals dienen. Der Betriebsgrenzwert beträgt beispielsweise +- 10g. Vorliegend wird durch einen Vergleich beider Messkanäle im eigentlichen Betrieb zusätzlich geprüft ob der Verstärkungsfaktor und der zugehörige Messwert bei der Gewichtsermittlung korrekt sind. Falls die Differenz zwischen beiden Gewichten zu groß ist, kann dies ein Indiz dafür sein, dass der Messkanal defekt ist.

Auf diese Weise kann die Verlässlichkeit der Vorrichtung vorteilhaft erhöht werden.

Gemäß einer bevorzugten Ausführungsform kann die Steuerungseinheit eingerichtet sein, über eine Lebensdauer der Vorrichtung hinweg einen weiteren erfassten ersten Initialisierungsgewichtsmesswert des ersten Messkanals mit vorherigen erfassten ersten Initialisierungsgewichtsmesswerten des ersten Messkanals hinsichtlich eines Trends auszuwerten und einen weiteren erfassten zweiten Initialisierungsgewichtsmesswert des zweiten Messkanals mit vorherigen erfassten zweiten Initialisierungsgewichtsmesswerten des zweiten Messkanals hinsichtlich eines Trends auszuwerten, wobei die Steuerungseinheit insbesondere eingerichtet ist, wenn ein Trend in dem ersten Messkanal und/oder dem zweiten Messkanal identifiziert worden ist, eine Fehlermeldung auszugeben.

Durch die Analyse der Initialisierungsgewichtsmesswerte hinsichtlich eines Trends können schleichende sich ändernde Bedingungen identifiziert werden und frühzeitig eine Fehlermeldung ausgegeben werden. Die Trends können auf ein baldiges Ausfallen der Vorrichtung hinweisen. Auf diese Weise kann die Verfügbarkeit der Vorrichtung vorteilhaft erhöht werden.

Ein weiterer Aspekt der vorliegenden Offenbarung betrifft ein computerimplementiertes Verfahren zum Betreiben einer Vorrichtung zum Wiegen einer Flüssigkeit, wobei die Vorrichtung eine Steuerungseinheit, eine Wägesensoreinheit mit einem ersten Messkanal und einem zweiten Messkanal, und eine Befestigungseinheit zum Befestigen eines Behälters umfasst, wobei das computerimplementierte Verfahren die Schritte umfasst: Erfassen jeweils vor dem Wiegen der Flüssigkeitsmenge in einem unbelasteten Zustand der Wägesensoreinheit einen ersten Initialisierungsgewichtsmesswert über den ersten Messkanal und einen zweiten Initialisierungsgewichtsmesswert über den zweiten Messkanal (S1); Vergleichen des erfassten ersten Initialisierungsgewichtsmesswert mit einem ersten Grenzwert und des zweiten Initialisierungsgewichtsmesswert mit einem zweiten Grenzwert (S2); Festlegen des erfassten ersten Initialisierungsgewichtsmesswert als Offset für den ersten Messkanal und des erfassten zweiten Initialisierungsgewichtsmesswert als Offset für den zweiten Messkanal, wenn der erste Initialisierungsgewichtsmesswert kleiner als der erste Grenzwert ist und der zweite Initialisierungsgewichtsmesswert kleiner als der zweite Grenzwert ist (S3); Ausgeben einer Fehlermeldung, wenn der erste Initialisierungsgewichtsmesswert größer als der erste Grenzwert ist und/oder der zweite Initialisierungsgewichtsmesswert größer als der zweite Grenzwert ist (S4).

Auf diese Weise kann zum einen die Messgenauigkeit erhöht werden, da jeweils mit einem genaueren Offset gearbeitet wird und zum anderen kann die Verfügbarkeit der Vorrichtung zum Wiegen einer Flüssigkeit erhöht werden, da eine Fehlermeldung bei einer schleichenden Verschlechterung durch eine neue Kalibrierung vermieden wird. Weiterhin ist es möglich, durch die beschriebene Kalibrierung vor jedem Messdurchlauf den ersten und/oder den zweiten Grenzwert größer festzulegen. Dies kann sich ebenfalls vorteilhaft auf die Verfügbarkeit der Vorrichtung auswirken.

Gemäß einer bevorzugten Ausführungsform kann die Fehlermeldung eine Aufforderung zur Überprüfung der Vorrichtung hinsichtlich von Messbedingungen umfassen.

Auf diese Weise kann die Verfügbarkeit der Vorrichtung vorteilhafterweise erhöht werden.

Gemäß einer bevorzugten Ausführungsform können nach der Überprüfung der Vorrichtung hinsichtlich der Messbedingungen die Schritte S1 bis S4 nochmals durchgeführt werden.

Auf diese Weise kann die Verfügbarkeit der Vorrichtung insgesamt erhöht werden, da die notwendige Inspektion eines Technikers mit neuer aufwändiger Kalibrierung umgangen werden kann.

Gemäß einer bevorzugten Ausführungsform kann ein Verfahren bereitgestellt werden, weiter umfassend: Vergleichen einer ersten Differenz zwischen dem ersten erfassten Initialisierungsgewichtsmesswert und einem ersten Ausgangsinitialisierungsgewichtsmesswert mit einem ersten Absolutgrenzwert; Vergleichen einer zweiten Differenz zwischen dem zweiten erfassten Initialisierungsgewichtsmesswert und einem zweiten Ausgangsinitialisierungsgewichtsmesswert mit einem zweiten Absolutgrenzwert; Festlegen des erfassten ersten Initialisierungsgewichtsmesswerts als Offset für den ersten Messkanal und des erfassten zweiten Initialisierungsgewichtsmesswerts als Offset für den zweiten Messkanal, wenn die erste Differenz kleiner als der erste Absolutgrenzwert und die zweite Differenz kleiner als der zweite Absolutgrenzwert ist; und Ausgeben einer Fehlermeldung, wenn die erste Differenz größer als der erste Absolutgrenzwert ist und/oder die zweite Differenz größer als der zweite Absolutgrenzwert ist.

Auf diese Weise kann auf synergetische Weise vorteilhaft ein Trend mildernd berücksichtigt werden, aber gleichzeitig eine zu große Veränderung der Messkanäle gegenüber dem Auslieferungszustand berücksichtigt werden. Dies kann die Verfügbarkeit bei gleichzeitiger Verlässlichkeit der Vorrichtung erhöhen.

Ein weiterer Aspekt der vorliegenden Offenbarung betrifft ein Computerprogrammprodukt, umfassend Befehle, die bei der Ausführung des Programms durch einen Computer oder Mikroprozessor diesen veranlassen, das oben näher beschriebene Verfahren auszuführen.

Ein weiterer Aspekt betrifft ein computerlesbares Speichermedium, umfassend Befehle, die bei der Ausführung durch einen Computer oder eine Mikroprozessor diesen veranlassen, das oben näher beschriebene Verfahren auszuführen.

Ein weiterer Aspekt betrifft eine extrakorporale Blutbehandlungsmaschine, insbesondere ein Dialysegerät, mit einer oben näher beschriebenen Vorrichtung.

Die Einheiten gemäß einem oder mehreren Ausführungsbeispielen können unter Verwendung von Hardware, Software und/oder einer Kombination davon implementiert werden. Die Einheiten können einteilig oder mehrteilig sein. Hardware-Einheiten können beispielsweise durch Verarbeitungsschaltungen wie einen Prozessor, eine Zentraleinheit (CPU), einen Controller, eine arithmetische Logikeinheit (ALU), einen digitalen Signalprozessor, einen Mikrocomputer, ein feldprogrammierbares Gate-Array (FPGA), ein System-on-Chip (SoC), eine programmierbare Logikeinheit, einen Mikroprozessor oder jede andere Vorrichtung implementiert werden, die in der Lage ist, auf Befehle zu reagieren und diese in einer festgelegten Weise auszuführen.

Die Einheiten können eine oder mehrere Schnittstellenschaltungen umfassen. In einigen Beispielen können die Schnittstellenschaltungen verdrahtete oder drahtlose Schnittstellen umfassen, die mit einem lokalen Netz (LAN), dem Internet, einem Weitverkehrsnetz (WAN) oder Kombinationen davon verbunden sind. Die Funktionalität einer bestimmten Einheit der vorliegenden Offenbarung kann auf mehrere Einheiten verteilt werden, die über Schnittstellenschaltungen verbunden sind.

Die Einheiten gemäß einem oder mehreren Ausführungsbeispielen können auch ein oder mehrere Speichergeräte umfassen. Bei der einen oder den mehreren Speichervorrichtungen kann es sich um materielle oder nichttransitorische computerlesbare Speichermedien handeln, wie Direktzugriffsspeicher (RAM), Festwertspeicher (ROM), ein permanentes Massenspeichergerät (z. B. ein Festplattenlaufwerk), ein Solid-State-Gerät (z. B. NAND Flash) und/oder jeder andere Datenspeichermechanismus, der Daten speichern und aufzeichnen kann. Die eine oder mehrere Speichervorrichtungen können zum Speichern von Computerprogrammen, Programmcode, Anweisungen oder eine Kombination davon eingerichtet sein.

Die hier beschriebenen Erläuterungen und Vorteile einzelner Ausführungsformen gelten sinngemäß auch für die anderen Ausführungsformen. Verschiedene beispielhafte Merkmale der Ausführungsformen können erfindungsgemäß kombiniert werden, wo immer dies technisch sinnvoll und machbar ist.

Kurzbeschreibung der Figuren
Fig. 1 ist eine Darstellung zur Veranschaulichung einer Vorrichtung zum Wiegen einer Flüssigkeit in einem Dialysegerät gemäß einem ersten Ausführungsbeispiel der vorliegenden Offenbarung;
Fig. 2 ist ein Diagramm eines Verlaufs eines Offsets über mehrere Messzyklen ohne Verwendung der offenbarungsgemäßen Vorrichtung;
Fig. 3 ist ein Diagramm eines Verlaufs eines Offsets über mehrere Messzyklen ohne Verwendung einer offenbarungsgemäßen Vorrichtung;
Fig. 4 ist ein Diagramm eines weiteren Verlaufs eines Offsets über mehrere Messzyklen unter Verwendung einer offenbarungsgemäßen Vorrichtung; und
Fig. 5 ist ein Diagramm mit einem Vergleich eines ersten Initialisierungsgewichtsmesswerts und eines zweiten Initialisierungsgewichtsmesswerts.

### Beschreibung der Ausführungsbeispiele

Nachstehend werden Ausführungsbeispiele der vorliegenden Offenbarung auf der Basis der zugehörigen Figuren beschrieben.

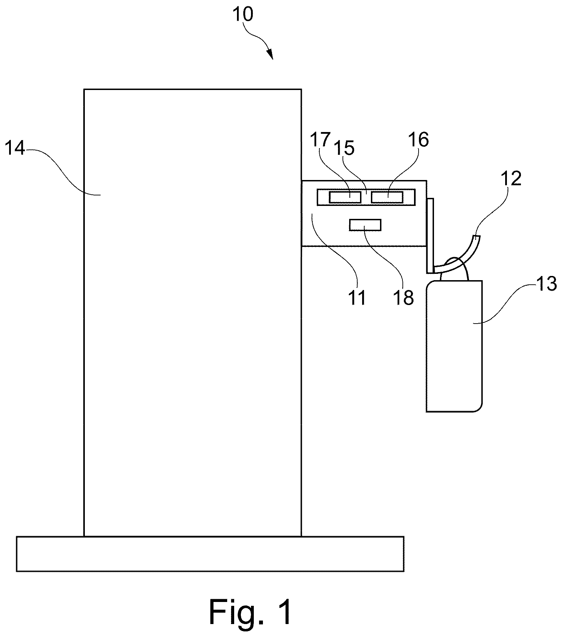

Figur 1 zeigt eine Vorrichtung 10 zum Wiegen einer Flüssigkeitsmenge in einem Dialysegerät 14. Die Flüssigkeitsmenge befindet sich vorliegend in einem Behälter 13. Aus diesem Behälter 13 fließt die Flüssigkeitsmenge beispielsweise in einen Patienten (nicht gezeigt) oder in eine Filtereinheit (nicht gezeigt) des Dialysegeräts 14. Alternativ kann in diesen Behälter 13 auch eine Flüssigkeitsmenge, beispielsweise verbrauchte Dialysierflüssigkeit/ Dialysat hineinfließen. Hierzu weist der Behälter 13 zumindest eine Öffnung (Einlassöffnung oder Auslassöffnung) auf. Die Vorrichtung 10 weist vorliegend eine Befestigungseinheit 11 zur Aufnahme des Behälters 13 auf. Die Befestigungseinheit 11 umfasst vorliegend als Schnittstelle zur Aufnahme des Behälters 13 einen Haken 12. Dieser kann beispielsweise formschlüssig, kraftschlüssig oder stoffschlüssig mit der Befestigungseinheit 11 verbunden sein. Die Befestigungseinheit 11 ist vorliegend mit einem Gestellelement 14 verbunden. Das Gestellelement 14 entspricht vorliegend dem Dialysegerät 14. Die Vorrichtung 10 umfasst weiterhin eine Wägesensoreinheit 15 mit zwei Messkanälen 16 und 17 zum Messen eines ersten Gewichtsmesswerts und zum Messen eines zweiten Gewichtsmesswertes. Die beiden Messkanäle messen dabei die Last, die an der Befestigungseinheit 11 und/oder am Haken 12 angreift. Die beiden Messkanäle weisen vorliegend jeweils DMS Sensoren, Hardware und Software zum Bestimmen der Gewichtsmesswerte auf. Die Messkanäle sind vorliegend identisch. Alternativ können sie sich auch unterscheiden. Die Vorrichtung 10 weist weiterhin ein Steuerungseinheit 18 auf. Die Steuerungseinheit 18 ist vorliegend in der Befestigungseinheit 11 angeordnet. Diese kann alternativ aber auch räumlich von dieser getrennt angeordnet sein, beispielsweise in dem Dialysegerät 14. Die Steuerungseinheit 18 ist eingerichtet, auf Basis des erfassten ersten Gewichtsmesswertes und des erfassten zweiten Gewichtsmesswertes ein Gewicht für die Flüssigkeitsmenge in dem Behälter 13 zu bestimmen. Die Steuerungseinheit 18 bestimmt vorliegend ein Gewicht für die Flüssigkeitsmenge in dem Behälter 13 dadurch, indem sie den ersten Gewichtsmesswert als Wert für das Gewicht für die Flüssigkeitsmenge in dem Behälter 13 verwendet. Die Steuerungseinheit 18 ist vorliegend weiterhin eingerichtet, eine Differenz zwischen dem ersten Gewichtsmesswert und dem zweiten Gewichtsmesswert zu bilden und diese mit einem Arbeitsgrenzwert zu vergleichen. Der Arbeitsgrenzwert beträgt vorliegend 10g. Die Steuerungseinheit 18 ist vorliegend weiterhin eingerichtet, wenn die Differenz den Arbeitsgrenzwert überschreitet, eine Fehlermeldung (z.B. zu hohe Messabweichung zwischen den Messkanälen) auszugeben. Die Steuerungseinheit 18 ist weiterhin eingerichtet, wenn die Differenz den Arbeitsgrenzmesswert nicht überschreitet, das Gewicht für die Flüssigkeitsmenge in dem Behälter 13 auszugeben. Die Wägesensoreinheit 15 ist eingerichtet, jeweils vor dem Wiegen der Flüssigkeitsmenge in einem unbelasteten Zustand der Wägesensoreinheit 15 einen ersten Initialisierungsgewichtsmesswert über den ersten Messkanal 16 zu erfassen und einen zweiten Initialisierungsgewichtsmesswert über den zweiten Messkanal 17 zu erfassen (S1). Im vorliegenden Fall ist die Wägesensoreinheit 15 unbelastet, wenn kein Behälter 13 am Haken 12 hängt. Die Steuerungseinheit 18 ist weiter eingerichtet, den erfassten ersten Initialisierungsgewichtsmesswert mit einem ersten Grenzwert zu vergleichen und den zweiten Initialisierungsgewichtsmesswert mit einem zweiten Grenzwert zu vergleichen (S2). Der erste Grenzwert und der zweite Grenzwert betragen vorliegend jeweils bevorzugt +- 20g. Weiterhin ist die Steuerungseinheit 18 eingerichtet, wenn der erste Initialisierungsgewichtsmesswert kleiner als der erste Grenzwert ist und der zweite Initialisierungsgewichtsmesswert kleiner als der zweite Grenzwert ist, den erfassten ersten Initialisierungsgewichtsmesswert als Offset für den ersten Messkanal festzulegen und den erfassten zweiten Initialisierungsgewichtsmesswert als Offset für den zweiten Messkanal festzulegen (S3). Vorliegend beträgt der erste erfasste Initialisierungsgewichtsmesswert beispielsweise 17g. Somit ist der erste Initialisierungsgewichtsmesswert kleiner als der erste Grenzwert, der +- 20g beträgt. Vorliegend beträgt der zweite erfasste Initialisierungsgewichtsmesswert beispielsweise 23g. Somit ist der zweite Initialisierungsgewichtsmesswert größer als der zweite Grenzwert, der +- 20g beträgt. Somit legt die Steuerungseinheit 18 vorliegend keine neuen Offsets für den ersten Messkanal und den zweiten Messkanal fest. Weiterhin ist die Steuerungseinheit 18 eingerichtet, wenn der erste Initialisierungsgewichtsmesswert größer als der erste Grenzwert ist und/oder der zweite Initialisierungsgewichtsmesswert größer als der zweite Grenzwert ist, eine Fehlermeldung auszugeben (S4). Vorliegend ist der zweite Initialisierungsgewichtsmesswert mit 23g größer als der zweite Grenzwert mit +- 20g. Somit gibt die Steuerungseinheit 18 eine Fehlermeldung aus. Die Fehlermeldung umfasst vorliegend eine Aufforderung zur Überprüfung der Vorrichtung hinsichtlich von Messbedingungen. Die Fehlermeldung wird vorliegend an einem Display der Vorrichtung 10 angezeigt. Die Fehlermeldung kann optional weiter beispielsweise eine Überprüfung des Hakens 12 oder der Vorrichtung 10 im Gesamten umfassen. Wären alternativ beide Initialisierungsgewichtsmesswerte jeweils kleiner als die entsprechenden Grenzwerte, würde die Steuerungseinheit 18 die beiden Initialisierungsgewichtsmesswerte jeweils als Offset für die jeweiligen Messkanäle verwenden, sodass beide Messkanäle im unbelasteten Zustand das Gewicht 0g anzeigen.

Weiterhin vergleicht die Steuerungseinheit 18 eine erste Differenz zwischen dem ersten erfassten Initialisierungsgewichtsmesswert und einem ersten Ausgangsinitialisierungsgewichtsmesswert mit einem ersten Absolutgrenzwert. Der erste Ausgangsinitialisierungsgewichtsmesswert wurde vorliegend bei der Herstellung der Vorrichtung ermittelt. Der erste Absolutgrenzwert beträgt vorliegend +-100g. Weiterhin vergleicht die Steuerungseinheit 18 eine zweite Differenz zwischen dem zweiten erfassten Initialisierungsgewichtsmesswert und einem zweiten Ausgangsinitialisierungsgewichtsmesswert mit einem zweiten Absolutgrenzwert. Der zweite Ausgangsinitialisierungsmesswert wurde vorliegend bei der Herstellung der Vorrichtung ermittelt. Der zweite Absolutgrenzwert beträgt vorliegend +-100g. Die Differenzen sind vorliegend jeweils kleiner als die Absolutgrenzwerte, sodass keine Fehlermeldung deswegen ausgegeben wird.

Weiterhin ermittelt die Steuerungseinheit 18, wenn der erste Initialisierungsgewichtsmesswert größer als der erste Grenzwert ist und der zweite Initialisierungsgewichtsmesswert größer als der zweite Grenzwert ist, eine Differenz des ersten Initialisierungsgewichtsmesswerts und des zweiten Initialisierungsgewichtsmesswerts und vergleicht diese Differenz mit einem Toleranzgrenzwert. Der Toleranzgrenzwert ist beispielsweise 5g. Wenn die Differenz kleiner als der Toleranzgrenzwert ist, gibt die Steuerungseinheit 18 eine Fehlermeldung mit einer Aufforderung zur Überprüfung der Messbedingungen aus. Vorliegend ist lediglich der zweite Initialisierungsgewichtsmesswert größer als der zweite Grenzwert, sodass diese Überprüfung nicht ausgeführt wird.

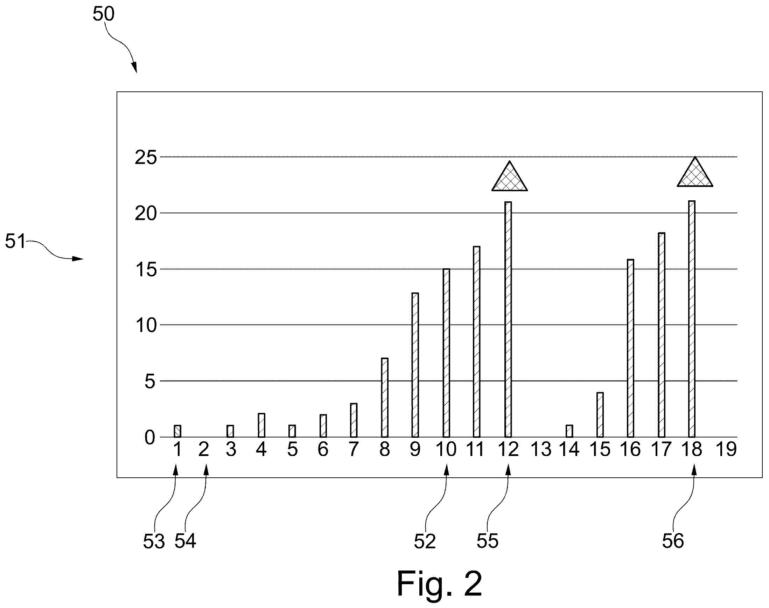

Figur 2 zeigt den Verlauf 50 des Offsets über eine Vielzahl an Messungen für eine Vorrichtung zum Wiegen einer Flüssigkeitsmenge, die nicht der offenbarungsgemäßen Vorrichtung entspricht. Auf der Vertikalachse 51 ist der Offset für einen unbelasteten Zustand der Vorrichtung aufgetragen. Auf der Horizontalachse 52 ist die Anzahl der Messungen mit der Vorrichtung aufgetragen. Bei der ersten Messung 53, beispielsweise im Produktionswerk, wird ein entsprechender Offset ermittelt und neu eingestellt, sodass die Vorrichtung bei der zweiten Messung 54 Null als Messwert anzeigt. Über die Messungen vergrößert sich vorliegend der Offset. Bei der zwölften Messung 55 überschreitet der Messwert im unbelasteten Zustand einen Grenzwert. Der Grenzwert beträgt beispielsweise 20g vorliegend. In diesem Fall, muss dann ein Techniker die Vorrichtung aufwändig kalibrieren (z. B. mit einem Messgewicht), sodass sie bei der dreizehnten Messung wieder den Wert Null als Messwert anzeigt. Danach steigt der Offset über die einzelnen Messungen wieder hinweg bis wiederum der Grenzwert bei der achtzehnten Messung 56 überschritten wird.

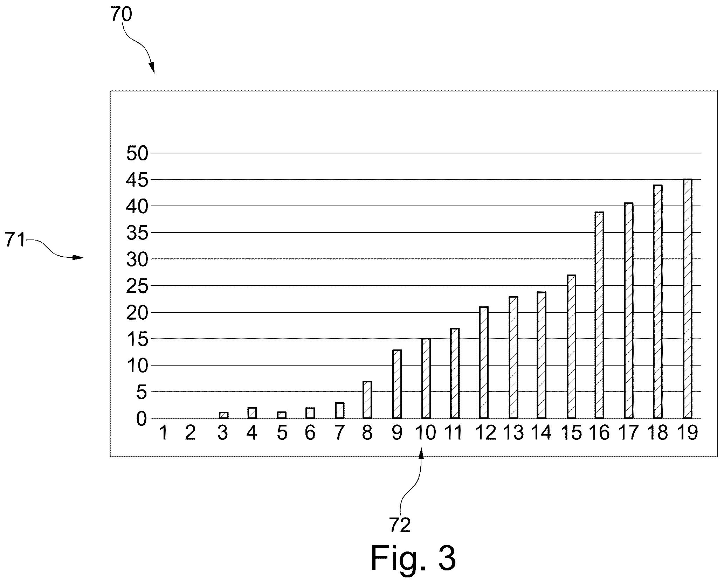

Figur 3 zeigt den Verlauf 70 des Offsets über eine Vielzahl an Messungen für eine weitere Vorrichtung zum Wiegen einer Flüssigkeitsmenge, die nicht der offenbarungsgemäßen Vorrichtung entspricht. Auf der Vertikalachse 71 ist der Offset für einen unbelasteten Zustand der Vorrichtung aufgetragen. Auf der Horizontalachse 72 ist die Anzahl der Messungen mit der Vorrichtung aufgetragen. Im Gegensatz zum Verlauf 50, wird hier keine Rekalibrierung bei Überschreiten eines Grenzwertes durch einen Techniker durchgeführt. Der Verlauf 70 steigt kontinuierlich an.

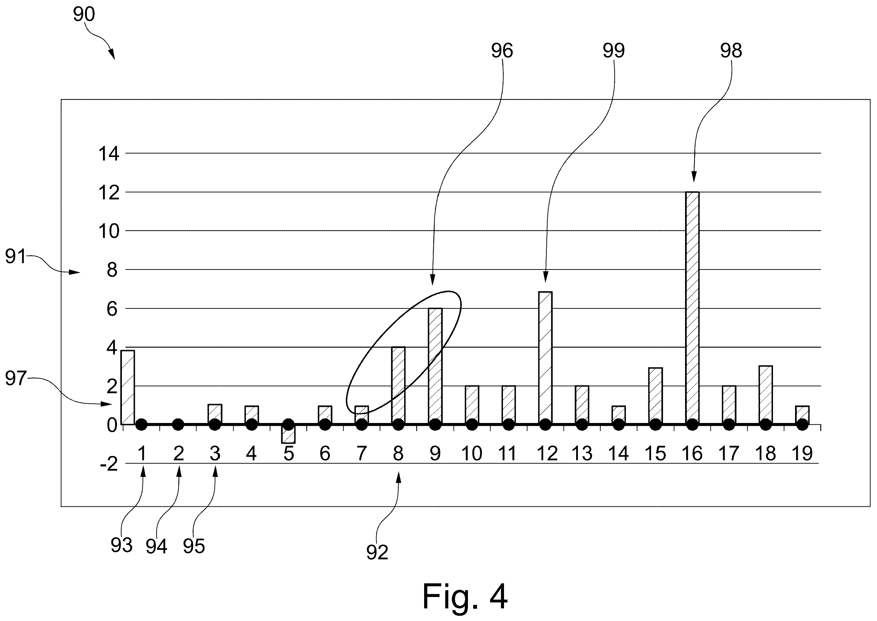

Figur 4 zeigt den Verlauf 90 des Offsets über eine Vielzahl an Messungen für eine offenbarungsgemäße Vorrichtung zum Wiegen einer Flüssigkeitsmenge. Auf der Vertikalachse 91 ist der Offset für einen unbelasteten Zustand der Vorrichtung 10 für den ersten Messkanal aufgetragen. An dieser Stelle sei erwähnt, dass die Ausführungen analog für den zweiten Messkanal gelten. Auf der Horizontalachse 92 ist die Anzahl der Messungen mit der Vorrichtung 10 aufgetragen. Hierbei zeigt sich, dass beispielsweise bei den beiden ersten Messungen 93, 94 keine neue Abweichung gemessen wurde, bei der dritten Messung 95 hingegen schon. Diese wurde dann als neuer Offset gewählt. Insgesamt ist zu sehen, dass vor jeder eigentlichen Messung der Offset neu ermittelt wird. Dies kann auch als Autokalibrierung bezeichnet werden.

Weiterhin ist im Bereich 96 zu erkennen, dass die Messungen überproportional ansteigen. Die Steuerungseinheit 18 wertet die jeweiligen Initialisierungsgewichtsmesswerte hinsichtlich eines solchen Anstiegs bzw. Trends aus, indem sie beispielsweise die Differenzen zwischen aufeinanderfolgender Initialisierungsgewichtsmesswerte bildet und diese miteinander ins Verhältnis setzt. Im Falle einer Ermittlung eines solchen Trends, gibt die Steuerungseinheit 18 eine entsprechende Fehlermeldung aus. Die Fehlermeldung kann beispielsweise eine Aufforderung zur Überprüfung der Umgebungsbedingungen beinhalten.

Weiterhin ist im Diagramm ein erster Ausgangsinitialisierungsgewichtsmesswert 97 eingezeichnet. Der erste Ausgangsinitialisierungsmesswert 97 weist vorliegend 4g auf. Beispielsweise beträgt der oben erwähnte erste Absolutgrenzwert 7g. Der erste Initialisierungsgewichtsmesswert 98 beträgt 12g. Eine zugehörige Differenz zwischen dem ersten Initialisierungsgewichtsmesswert 98 und dem ersten Ausgangsinitialisierungsgewichtsmesswert beträgt vorliegend 8g und ist somit größer als erste Absolutgrenzwert von 7g. In diesem Fall würde eine Fehlermeldung ausgegeben werden, die eine Hinzuziehung eines Technikers zur Neukalibrierung der Vorrichtung beinhaltet.

Weiterhin beträgt beispielsweise der erste Grenzwert vorliegend 6g. Der Messwert 99 würde vorliegend den ersten Grenzwert von 6 überschreiten, sodass eine Fehlermeldung ausgegeben wird.

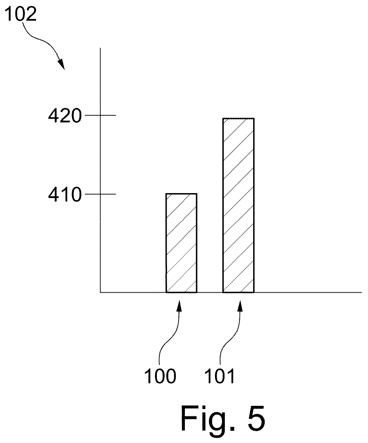

Figur 5 zeigt einen Vergleich eines Gewichts 100 des ersten Messkanals 16 und eines Gewichts 101 des zweiten Messkanals 17, die während dem Betrieb der Vorrichtung 10 ermittelt wurden. Auf der Vertikalachse 102 sind die Werte für die Gewichte aufgetragen. Die Differenz der beiden Gewichte beträgt vorliegend 10g. Die Differenz ist somit vorliegend kleiner als der Betriebsgrenzwert von 20g. Somit gibt die Steuerungseinheit 18 keine Fehlermeldung aus. Andernfalls, würde die Steuerungseinheit 18 eine Fehlermeldung ausgeben.

### Bezugszeichenliste

- 10: Vorrichtung
- 11: Befestigungseinheit
- 12: Haken
- 13: Behälter
- 14: Gestellelement, Dialysegerät
- 15: Wägesensoreinheit
- 16: erster Messkanal
- 17: zweiter Messkanal
- 18: Steuerungseinheit
- 50, 70, 90: Verlauf Offset
- 51, 71, 91, 102: Vertikalachse
- 52, 72, 92: Horizontalachse
- 53, 54, 55, 56: einzelne Messungen ohne mechanische Belastung
- 93, 94, 95: einzelne Messung mit anschließender Autokalibrierung
- 96: Bereich mit Trend
- 97: Ausgangsinitialisierungsgewichtsmesswert
- 98: einzelne Messung größer als Grenzwert
- 99: Messung
- 101: Gewicht erster Messkanal
- 102: Gewicht zweiter Messkanal

## Patentansprüche

1. Vorrichtung (10) zum Wiegen einer Flüssigkeitsmenge in einer extrakorporalen Blutbehandlungsmaschine, umfassend:
eine Befestigungseinheit (11), die eingerichtet ist, einen Behälter (13) für die Flüssigkeitsmenge an einem Gestellelement (14) zu befestigen;
eine Wägesensoreinheit (15) mit einem ersten Messkanal (16) und einem zweiten Messkanal (17), wobei die Wägesensoreinheit (15) eingerichtet ist, einen ersten Gewichtsmesswert über den ersten Messkanal (16) zu erfassen und einen zweiten Gewichtsmesswert über den zweiten Messkanal (17) zu erfassen;
eine Steuerungseinheit (18), die eingerichtet ist, auf Basis des erfassten ersten Gewichtsmesswertes und des erfassten zweiten Gewichtsmesswertes ein Gewicht für die Flüssigkeitsmenge in dem Behälter (13) zu bestimmen;
wobei die Wägesensoreinheit (15) eingerichtet ist, jeweils vor dem Wiegen der Flüssigkeitsmenge in einem unbelasteten Zustand der Wägesensoreinheit (15) einen ersten Initialisierungsgewichtsmesswert über den ersten Messkanal (16) zu erfassen und einen zweiten Initialisierungsgewichtsmesswert über den zweiten Messkanal (17) zu erfassen (S1);
wobei die Steuerungseinheit (18) eingerichtet ist, den erfassten ersten Initialisierungsgewichtsmesswert mit einem ersten Grenzwert zu vergleichen und den zweiten Initialisierungsgewichtsmesswert mit einem zweiten Grenzwert zu vergleichen (S2);
wobei die Steuerungseinheit (18) eingerichtet ist:
wenn ein Betrag des ersten Initialisierungsgewichtsmesswerts kleiner als ein Betrag des ersten Grenzwerts ist und ein Betrag des zweiten Initialisierungsgewichtsmesswerts kleiner als ein Betrag des zweiten Grenzwerts ist, den erfassten ersten Initialisierungsgewichtsmesswert als Offset für den ersten Messkanal (16) festzulegen und den erfassten zweiten Initialisierungsgewichtsmesswert als Offset für den zweiten Messkanal (17) festzulegen (S3); und
wenn ein Betrag des ersten Initialisierungsgewichtsmesswerts größer als ein Betrag des ersten Grenzwerts ist und/oder ein Betrag des zweiten Initialisierungsgewichtsmesswerts größer als ein Betrag des zweiten Grenzwerts ist, eine Fehlermeldung auszugeben (S4).

2. Vorrichtung (10) nach Anspruch 1, wobei die Fehlermeldung eine Aufforderung zur Überprüfung der Vorrichtung (10) hinsichtlich von Messbedingungen umfasst.

3. Vorrichtung (10) nach Anspruch 2, wobei nach der Überprüfung der Vorrichtung (10) hinsichtlich der Messbedingungen, die Vorrichtung (10) eingerichtet ist, die Schritte S1 bis S4 nochmals durchzuführen.

4. Vorrichtung (10) nach einem der Ansprüche 1 bis 3, wobei die Steuerungseinheit (18) weiter eingerichtet ist,
eine erste Differenz zwischen dem ersten erfassten Initialisierungsgewichtsmesswert und einem ersten Ausgangsinitialisierungsgewichtsmesswert mit einem ersten Absolutgrenzwert zu vergleichen,
eine zweite Differenz zwischen dem zweiten erfassten Initialisierungsgewichtsmesswert und einem zweiten Ausgangsinitialisierungsgewichtsmesswert mit einem zweiten Absolutgrenzwert zu vergleichen,
wenn die erste Differenz kleiner als der erste Absolutgrenzwert und die zweite Differenz kleiner als der zweite Absolutgrenzwert ist, den erfassten ersten Initialisierungsgewichtsmesswert als Offset für den ersten Messkanal (16) festzulegen und den erfassten zweiten Initialisierungsgewichtsmesswert als Offset für den zweiten Messkanal (17) festzulegen,
wenn die erste Differenz größer als der erste Absolutgrenzwert ist und/oder die zweite Differenz größer als der zweite Absolutgrenzwert ist, eine Fehlermeldung auszugeben.

5. Vorrichtung (10) nach einem der Ansprüche 1 bis 4, wobei die Steuerungseinheit (18) weiter eingerichtet ist, wenn der erste Initialisierungsgewichtsmesswert größer als der erste Grenzwert ist und der zweite Initialisierungsgewichtsmesswert größer als der zweite Grenzwert ist, und wenn eine Differenz des ersten Initialisierungsgewichtsmesswerts und des zweiten Initialisierungsgewichtsmesswerts kleiner als ein Toleranzgrenzwert ist, eine Fehlermeldung mit einer Aufforderung zur Überprüfung der Messbedingungen auszugeben.

6. Vorrichtung (10) nach einem der Ansprüche 1 bis 5, wobei die Steuerungseinheit (18) weiter eingerichtet ist, während dem Betrieb eine Differenz des mit dem ersten Messkanal (16) bestimmten Gewichts und des mit dem zweiten Messkanal (17) bestimmten Gewichts mit einem Betriebsgrenzwert zu vergleichen, und wenn die Differenz größer als der Betriebsgrenzwert ist, eine Fehlermeldung auszugeben.

7. Vorrichtung (10) nach einem der Ansprüche 1 bis 6, wobei die Steuerungseinheit (18) eingerichtet ist, über eine Lebensdauer der Vorrichtung (10) hinweg einen weiteren erfassten ersten Initialisierungsgewichtsmesswert des ersten Messkanals (16) mit vorherigen erfassten ersten Initialisierungsgewichtsmesswerten des ersten Messkanals (16) hinsichtlich eines Trends auszuwerten und einen weiteren erfassten zweiten Initialisierungsgewichtsmesswert des zweiten Messkanals (17) mit vorherigen erfassten zweiten Initialisierungsgewichtsmesswerten des zweiten Messkanals (17) hinsichtlich eines Trends auszuwerten, wobei die Steuereinrichtung insbesondere eingerichtet ist, wenn ein Trend in dem ersten Messkanal (16) und/oder zweiten Messkanal (17) identifiziert worden ist, eine Fehlermeldung auszugeben.

8. Computer implementiertes Verfahren zum Betreiben einer Vorrichtung (10) zum Wiegen einer Flüssigkeit, wobei die Vorrichtung (10) eine Steuerungseinheit (18), eine Wägesensoreinheit (15) mit einem ersten Messkanal (16) und einem zweiten Messkanal (17), und eine Befestigungseinheit (11) zum Befestigen eines Behälters (13) umfasst, wobei das Verfahren die Schritte umfasst:
Erfassen jeweils vor dem Wiegen der Flüssigkeitsmenge in einem unbelasteten Zustand der Wägesensoreinheit (15) einen ersten Initialisierungsgewichtsmesswert über den ersten Messkanal und einen zweiten Initialisierungsgewichtsmesswert über den zweiten Messkanal (S1);
Vergleichen des erfassten ersten Initialisierungsgewichtsmesswert mit einem ersten Grenzwert und des zweiten Initialisierungsgewichtsmesswert mit einem zweiten Grenzwert (S2);
Festlegen des erfassten ersten Initialisierungsgewichtsmesswerts als Offset für den ersten Messkanal (16) und des erfassten zweiten Initialisierungsgewichtsmesswerts als Offset für den zweiten Messkanal (17), wenn der erste Initialisierungsgewichtsmesswert kleiner als der erste Grenzwert ist und der zweite Initialisierungsgewichtsmesswert kleiner als der zweite Grenzwert ist (S3);
Ausgeben einer Fehlermeldung, wenn der erste Initialisierungsgewichtsmesswert größer als der erste Grenzwert ist und/oder der zweite Initialisierungsgewichtsmesswert größer als der zweite Grenzwert ist (S4).

9. Verfahren nach Anspruch 8, wobei die Fehlermeldung eine Aufforderung zur Überprüfung der Vorrichtung (10) hinsichtlich von Messbedingungen umfasst.

10. Verfahren nach Anspruch 8 oder 9, wobei nach der Überprüfung der Vorrichtung (10) hinsichtlich der Messbedingungen, die Vorrichtung (10) eingerichtet ist, die Schritte S1 bis S4 nochmals durchzuführen.

11. Verfahren nach einem der Ansprüche 8 bis 10, weiter umfassend die Schritte:
Vergleichen einer ersten Differenz zwischen dem ersten erfassten Initialisierungsgewichtsmesswert und einem ersten Ausgangsinitialisierungsgewichtsmesswert mit einem ersten Absolutgrenzwert,
Vergleichen einer zweiten Differenz zwischen dem zweiten erfassten Initialisierungsgewichtsmesswert und einem zweiten Ausgangsinitialisierungsgewichtsmesswert mit einem zweiten Absolutgrenzwert,
Festlegen des erfassten ersten Initialisierungsgewichtsmesswerts als Offset für den ersten Messkanal (16) und des erfassten zweiten Initialisierungsgewichtsmesswerts als Offset für den zweiten Messkanal (17), wenn die erste Differenz kleiner als der erste Absolutgrenzwert und die zweite Differenz kleiner als der zweite Absolutgrenzwert ist;
Ausgeben einer Fehlermeldung, wenn die erste Differenz größer als der erste Absolutgrenzwert ist und/oder die zweite Differenz größer als der zweite Absolutgrenzwert ist.

12. Computerprogrammprodukt, umfassend Befehle, die bei der Ausführung des Programms durch einen Computer oder Mikroprozessor diesen veranlassen, das Verfahren nach einem der Ansprüche 8 bis 11 auszuführen.

13. Computerlesbares Speichermedium, umfassend Befehle, die bei der Ausführung durch einen Computer oder einen Mikroprozessor diesen veranlassen, das Verfahren nach einem der Ansprüche 8 bis 11 auszuführen.

14. Extrakorporale Blutbehandlungsmaschine (14) mit einer Vorrichtung (10) nach einem der Ansprüche 1 bis 7.
